# EUROPEAN PATENT APPLICATION

(11) **EP 4 393 482 A1**
(43) Date of publication of application: **03.07.2024**
(21) Application number: 22861611.6
(22) Date of filing: 12.08.2022
(51) Int. Cl.: A61K 9/16, A61K 9/00, A61J 3/00, A61J 3/02, B01J 2/06, B01J 19/18

(54) **PARALLEL-TYPE MEMBRANE EMULSIFICATION METHOD AND DEVICE FOR PREPARING BIODEGRADABLE POLYMER MICROSPHERES, AND INJECTION FORMULATION PREPARATION METHOD USING SAME**

(30) Priority: 26.08.2021 KR 20210113132
(71) Applicant: Jeong, Minwook, Suwon-si, Gyeonggi-do 16582 (KR)
(72) Inventor: Jeong, Minwook, Suwon-si, Gyeonggi-do 16582 (KR)
(74) Representative: Heyerhoff Geiger GmbH & Co. KG
(86) International application number: PCT/KR2022/012123
(87) International publication number: WO 2023/027401

(57) **Abstract**

The present invention relates to a method and device for preparing biodegradable polymer microspheres, and an injection formulation preparation method using same and, more specifically, to a parallel-type continuous reaction membrane emulsification method and device which can not only adjust the size but also mass produce biodegradable polymer microspheres maintaining a uniform size while maintaining the spherical shape, and a method for preparing a microsphere injection formulation composition comprising same. According to the parallel-type continuous reaction membrane emulsification method and device for preparing biodegradable polymer microspheres according to one embodiment of the present invention, since the particle size of the biodegradable polymer microspheres can easily be adjusted, and also spherical particles of a uniform size can easily be prepared, the method and device are suitable for mass production. According to the injection formulation preparation method according to one embodiment of the present invention, the biodegradable polymer microspheres, which have a low injection pressure due to uniform particle size distribution and are optimized for drug encapsulation due to enhanced mechanical strength, may be utilized for an injection formulation for drugs or medical devices.

## Description

### [Technical Field]

The present invention relates to a method and device for preparing biodegradable polymer microspheres, and an injection formulation preparation method using the same and, more specifically, to a parallel-type continuous reaction membrane emulsification method and device which can not only adjust the size but also mass produce biodegradable polymer microspheres maintaining a uniform size while maintaining the spherical shape, and a method for preparing a microsphere injection formulation composition including the same.

### [Background Art]

A technology for preparing microspheres with regard to biodegradable polymers is a technology which has drawn keen attention not only from fields of plastic surgery and skin beauty, but also from fields of long-lasting sustained-release drug delivery through drug encapsulation [Heller, J. et al., Controlled release of water-soluble macromolecules from bioerodible hydrogels, Biomaterials, 4, 262-266, 1983; Langer, R., New methods of drug delivery, Science, 249, 1527-1533, 1990; Langer, R., Chem. Eng. Commun., 6, 1-48, 1980; Langer, R. S. and Peppas, N. A., Biomaterials, 2, 201-214, 1981; Heller, J., CRC Crit. Rev. Ther. Drug Cattrier Syst., 1(1), 39-90, 1984; Holland, S.J. Tighe, B. J. and Gould, P. L., J. Controlled Release, 155-180, 1986].

Recently, fillers using biodegradable polymer microspheres and depot formulations through drug encapsulation have been developed, but there are various problems in use due to non-uniform particle shape and size.

First of all, since the particle size is not regular, a thick injection needle is required to prevent clogging, and thus patient compliance is low, an injection pressure is high, and a pressure during injection is not regular, thus making it less convenient for doctors.

In addition, when a drug is encapsulated, an initial burst effect of the drug occurs due to a non-uniform thickness of a shell, a release rate of the drug is not controlled at a regular rate for a certain period of time, and it is difficult to control the regular release rate in vivo due to an incomplete release, in which the drug is not released 100% [Crotts, G. and Park, T.G., J. Control. Release, 44, 123-134, 1997; Leonard, N.B., Michael, L. H., Lee, M.M. J. Pharm. Sci., 84, 707-712].

Phase separation, spray-drying, emulsification solvent evaporation method, and micro-fluidic method are known as general methods for preparing microspheres currently used.

In the case of the phase separation method [Korean Patent Registration No. 10-1105292], a spray dryer is basically used, and thus it is difficult to prepare particles having a uniform size and the particles show porosity. Thus, the method is not suitable as the technology for encapsulating drugs which need to control a regular release rate.

Since the spray drying method [Korean Patent Registration No. 10-0566573] uses a large amount of solvent and exhibits porosity, the method is not suitable as a technology for preparing particles having a uniform size and encapsulating drugs. In addition, in some cases, a spray drying process is required at a high temperature of 60°C or higher, thus causing denaturation of biodegradable polymers weak to heat as well as encapsulated drugs.

Currently, the most commonly used method is the emulsification solvent evaporation method [Korean Patent Registration No. 10-2089560]. The method is a method for forming microspheres by strongly stirring a dispersion in which polymers are dissolved in an organic solvent and an emulsion including a surfactant. An emulsion is in a thermodynamically unstable state, and thus an aqueous phase and an organic phase tend to be separated from each other through a process such as coalescence, fusion, phase separation (creaming), etc. In order to prevent the separation, a strong stirring force is required, and thus there is a disadvantage in that mass synthesis is difficult by a general batch reaction, and there is also a disadvantage in that a spherical microsphere may be formed, but fundamentally uniform particles may not be formed.

The micro-fluidic method [Korean Patent Registration No. 10-2101969] is a technology which has recently drawn much attention due to an advantage of being able to make particles having a uniform size and a perfect sphere. However, since two micro control pumps need to be connected perpendicular to hundreds of thousands of microtubes for mass production, there is a problem in that equipment costs are very high and each microtube needs to be precisely controlled technically. In addition, in order to change a size of microspheres, there is a disadvantage in that thousands of bundles of microtubes need to be changed as a whole.

Thus, when preparing microspheres for tissue regeneration or drug encapsulation, there is a need for a method for preparing microspheres, which have a regular size and may be easily adjusted in size according to uses, and in which the preparation method is simple, economical, easy to mass-produce, have high mechanical strength and density, and thus have drug encapsulation and a regular release rate.

### [Related Art Reference]

### [Patent Documents]

(Patent Document 1) Korean Patent No. 10-1105292
(Patent Document 2) Korean Patent No. 10-0566573
(Patent Document 3) Korean Patent No. 10-2089560
(Patent Document 4) Korean Patent No. 10-2101969

### [Disclosure]

### [Technical Problem]

An object of the present invention is to provide a method for preparing microspheres and a device for preparing microspheres for mass-production, which are capable of easily adjusting a size of particles while regularly maintaining a shape and size of biodegradable polymer microspheres, and which may enable easy preparation and mass production with economical feasibility. In addition, another object of the present invention is to provide a use of the biodegradable polymer microspheres prepared by the above-described method for skin tissue reconstruction and tissue regeneration or as an injection formulation for medical and pharmaceutical uses through drug encapsulation.

### [Technical Solution]

As a method for preparing biodegradable polymer microspheres for solving the above-described objects, the present invention may provide a method for preparing biodegradable polymer microspheres using a parallel-type continuous reaction membrane emulsification device, the method including: preparing a dispersion in which biodegradable polymers are dissolved; applying a pressure to the dispersion and slowly injecting the dispersion into an emulsion through a parallel-type membrane to form microspheres; discharging a mixed solution in which the microspheres are mixed; receiving the discharged mixed solution to stabilize the microspheres; and washing and drying the stabilized microspheres, in which the emulsion is continuously replaced and supplied.

*In addition, as a parallel-type continuous reaction membrane emulsification device for preparing the polymer microspheres, the present invention may provide a membrane emulsification device using a parallel-type membrane and a continuous-type reaction, the device including: a dispersion tank for storing a dispersion; a pressurizing tank and a pressurizing line for pressurizing the dispersion; an emulsion tank provided with a membrane emulsification module and configured to receive the dispersion; an emulsion continuous supply line for continuously supplying an emulsion to the emulsion tank; a discharge port line for discharging a mixture of the dispersion and the emulsion to a stabilization tank; a stabilization tank for stabilizing microspheres through decompression or evaporation; and a manipulation part, in which the membrane emulsification module is a parallel-type membrane emulsification module in which membranes for injecting the dispersion into the emulsion are arranged in parallel to each other.

According to one embodiment of the present invention, the biodegradable polymer may be selected from polylactic acid (PLA) and isomers thereof, polyglycolic acid (PGA), polycarprolactone (PCL), polydioxanone (PDO), polylactic acid-glycolic acid (PLGA) and isomers thereof, etc., and an average molecular weight of the polymer may be 20,000 to 300,000.

According to one embodiment of the present invention, a dispersion in which the biodegradable polymers are dissolved may include a solvent. The solvent may include at least one of chlorinated hydrocarbon, hydrocarbon, perfluoroalcohol, ethyl ester (EA), ether-based solvent, N,N-dimethyl formamide (DMF), and dimethyl sulfoxide (DMSO).

According to one embodiment of the present invention, a content of the biodegradable polymer in the dispersion may be 1 to 20 wt%.

According to one embodiment of the present invention, the dispersion tank may be sealed and include an adjustment device capable of finely adjusting a pressure from the pressurizing tank, in which the pressure from the pressurizing tank may be adjusted to 1 kPa to 300 kPa according to a pore size of a membrane.

The membranes arranged in parallel to each other in the membrane emulsification module may be configured such that a plurality of small membranes are mounted in parallel to each other or a plurality of small reactors respectively equipped with one membrane are provided in parallel with each other. In addition, a size of the biodegradable polymer microparticles may be easily adjusted within 1 to 300 µm by replacing with a membrane having a different pore.

According to one embodiment of the present invention, an emulsion continuous supply device for continuously injecting a new emulsion may be included to maintain a mixture of the dispersion and the emulsion at a regular concentration, when the dispersion is slowly injected into the emulsion through the membrane of the membrane emulsification module, and a stirring device may be included to evenly disperse the mixed solution. In this case, an amount of the emulsion added may be 1 to 20 times that of the dispersion.

According to one embodiment of the present invention, the emulsion may include at least one of polyvinyl alcohol, polyoxyethylene sorbitan and salts thereof, soybean lecithin, and monoglyceride.

According to one embodiment of the present invention, the discharge port line may include a discharge port at a top of the emulsion tank which discharges an overflowing mixed solution when the emulsion is continuously injected, or may include a fluid pump.

According to one embodiment of the present invention, in the stabilizing of the microspheres, natural evaporation or vacuum evaporation through stirring may be performed.

A stabilization liquid used in the stabilizing may include at least one of polyvinyl alcohol, polyoxyethylene sorbitan and salts thereof, soybean lecithin, and monoglyceride.

According to one embodiment of the present invention, in the washing of the microspheres, the microspheres may be separated by a solid-liquid separation method of precipitating the microspheres and removing an upper solution, and then washing may be repeated several times using a washing liquid, and the washing liquid may include at least one of alkyl alcohol, distilled water, or a mixed solution of alkyl alcohol and distilled water.

According to one embodiment of the present invention, a freeze-drying method may be used to evenly spread a cake of microspheres containing moisture in the drying, and freeze and dry the same. Alternatively, if the microspheres are made of a highly heat-resistant biodegradable polymer material, the microspheres may be dried over dry heat or naturally.

The present invention may also provide an injection formulation including microspheres prepared according to the present invention.

According to one embodiment of the present invention, there may be included: preparing an aqueous solution including biodegradable polymer microspheres prepared according to the present invention; injecting the aqueous solution into a vial or a syringe; cooling and freezing the injected aqueous solution, followed by freeze-drying; and sealing and sterilizing the vial or the syringe.

In the preparing of the aqueous solution including biodegradable polymer microspheres, a content of polymers mixed in the aqueous solution may be 1 to 10 wt%.

In addition, in the preparing of the aqueous solution including biodegradable polymer microspheres, the aqueous solution may further include an additive, and the additive may include at least one of carboxymethyl cellulose and salts thereof, alginic acid and salts thereof, hyaluronic acid and salts thereof, dextran and salts thereof, collagen, gelatin, and elastin, and a content of the additive may be 1 to 30 wt% based on the total weight of the aqueous solution.

In the injecting of the aqueous solution into a vial or a syringe, the aqueous solution may be injected into a vial or syringe to be cooled and then freeze-dried. In addition, when filled in a vial or syringe in a powder state, the aqueous solution including the biodegradable polymer microspheres may be cooled, freeze-dried, pulverized into a powder, and filled.

In the cooling and freezing of the injected mixed solution, followed by freeze-drying, the filled vial or syringe may be cooled, and then dry-freezed. In addition, when filled in a powder state, the aqueous solution including the biodegradable polymer microspheres may be cooled, freeze-dried, pulverized into a powder, and filled.

In the sealing and sterilizing of the vial or the syringe, the sterilization method may be performed through gamma-ray sterilization, e-beam sterilization, ethylene oxide sterilization, or vacuum sterilization.

The injection formulation may be used as a face forming filler for skin tissue reconstruction and tissue regeneration, a male prosthesis, or a therapeutic agent for urinary incontinence treatment, or may be used as a long-lasting injection formulation for medical and pharmaceutical uses through drug encapsulation.

### [Advantageous Effects]

According to a parallel-type continuous reaction membrane emulsification method and device for preparing biodegradable polymer microspheres according to one embodiment of the present invention, a particle size of the biodegradable polymer microspheres can be easily adjusted, and also spherical particles having a uniform size can be easily prepared, and thus the method and device are suitable for mass production.

According to the injection formulation preparation method according to one embodiment of the present invention, the biodegradable polymer microspheres, which have a low injection pressure due to uniform particle size distribution and are optimized for drug encapsulation due to enhanced mechanical strength, can be utilized in an injection formulation for drugs or medical devices.

### [Description of Drawings]

FIG. 1 is a flow chart schematically showing a method for preparing biodegradable polymer microspheres according to one embodiment of the present invention.
FIG. 2 is a flow chart schematically showing a method for preparing an injection formulation according to one embodiment of the present invention.
FIG. 3 is a conceptual view schematically showing a parallel-type continuous reaction membrane emulsification device for preparing biodegradable polymer microspheres according to one embodiment of the present invention.
FIG. 4 is a schematic view showing a key device of a parallel-type continuous reaction membrane emulsification device for preparing biodegradable polymer microspheres according to one embodiment of the present invention.
FIG. 5 is an enlarged optical micrograph of the general biodegradable polymer microspheres of Examples 1 to 3.
FIG. 6 is an optical micrograph of biodegradable polymer microspheres obtained by the parallel-type continuous reaction membrane emulsification method of Examples 1 to 3 and an emulsification solvent evaporation method.
FIG. 7 is an optical micrograph of microspheres made to have a desired size using membranes having different pore sizes.

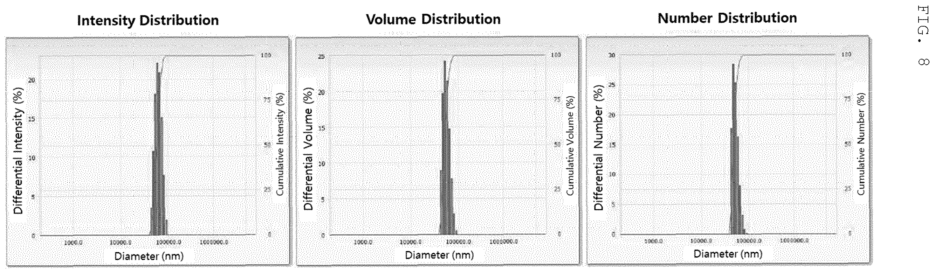

FIG. 8 is a particle size distribution graph of microspheres made according to one embodiment of the present invention.

### Best Mode

Hereinafter, Examples of the present invention will be described in detail so that those skilled in the art may easily carry out the present invention. However, the present invention may be implemented in various different forms and is not limited to the embodiments described herein.

First, a parallel-type continuous reaction membrane emulsification method and device for preparing biodegradable polymeric microspheres, and a method for preparing an injection formulation including the prepared microspheres will be described.

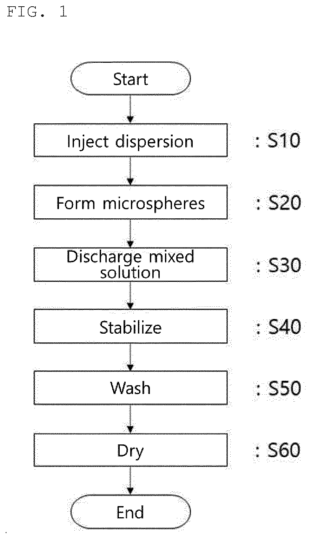

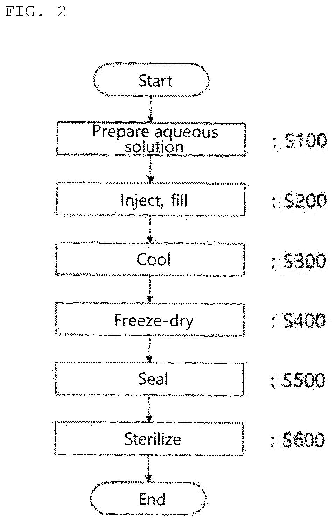

FIG. 1 is a flow chart schematically showing a method for preparing biodegradable polymer microspheres according to one embodiment of the present invention. In addition, FIG. 2 is a flow chart schematically showing a method for preparing an injection formulation according to one embodiment of the present invention.

Referring to FIG. 1, the preparing of biodegradable polymer microspheres may include injecting a dispersion in which biodegradable polymers are dispersed into a parallel-type continuous reaction membrane emulsification device (S10), slowly forming microspheres in the dispersion through a parallel-type membrane (S20), discharging a mixed solution while continuously flowing an emulsion (S30), stabilizing the microspheres formed in the collected discharged liquid (S40), washing the formed microspheres (S50), and drying to completely remove moisture (S60).

S10: First, inject a dispersion in which biodegradable polymers are dispersed into a dispersion tank.

The biodegradable polymers used may be selected from polylactic acid (PLA) and isomers thereof, polyglycolic acid (PGA), polycarprolactone (PCL), polydioxanone (PDO), and poly lactic acid-glycolic acid (PLGA) and isomers thereof.

Polydioxanone may be dissolved in perfluoroalcohol. Perfluoroalcohol may be an alcohol compound having one to six carbon atoms in which three to 13 fluorine atoms are substituted, and may include, for example, 1,1,1,3,3,3-hexafluoro-2-propanol.

An average molecular weight of the biodegradable polymer may be 20,000 to 300,000. When the average molecular weight of the biodegradable polymer is less than 20,000, a degradation rate may be fast, and thus a value as a material for tissue repair, tissue regeneration or drug delivery may decrease, and when the average molecular weight of the biodegradable polymer exceeds 300,000, it is difficult to process due to high viscoelasticity, thus making it difficult to make microspheres having a uniform size and quality.

A content of the biodegradable polymer may be 1 to 20 wt% based on the dispersion. When the content of the biodegradable polymer is less than 1%, the formed microspheres may not be suitable for drug encapsulation due to weak strength, and when the content is more than 20%, the formed microspheres may not pass through a membrane due to high viscosity or may not be suitable for mass-production because a high pressure is required.

The dispersion may include a solvent. The solvent may include at least one of chlorinated hydrocarbon, hydrocarbon, perfluoroalcohol, ethyl ester (EA), ether-based solvent, N,N-dimethyl formamide (DMF), and dimethyl sulfoxide (DMSO).

S20: Form biodegradable polymer microspheres while coming into contact with the emulsion when the dispersion is pressurized and slowly passed through a parallel-type membrane.

When the sealed dispersion tank is pressurized with air or nitrogen gas to push the dispersion and slowly pass through the parallel-type membrane, the dispersion may come into contact with the emulsion, thereby causing emulsification. In this case, the pressure applied according to a pore size of the membrane may be 1 kPa to 300 kPa. The smaller the pore of the membrane, the higher the pressure may be required, and the larger the pore of the membrane, the smaller the pressure value may be provided.

At this time, the biodegradable polymer microspheres may be formed while emulsification occurs at the same time. This is a method using a membrane emulsification method rather than a general mechanical stirring method, for example, a stirring method using a magnetic bar, or a stirring method using a mechanical stirrer or a homogenizer. Compared to a batch-type stirring method, the membrane emulsification method may have an advantage in that a size of microspheres may be adjusted, microspheres having a regular size may be made, and accurate spherical particles may be made. However, a small amount may be made only through a small-sized membrane, and when the content of the polymer in the dispersion is increased, the membrane may be clogged or the microspheres stick together.

The present invention relates to equipment for preparing microspheres with introduction of a parallel-type membrane arrangement and a continuous reaction concept, in which the shortcomings of a conventional single-channel batch-type membrane emulsification device are ameliorated to have an efficiency of 100 times or higher based on a single production amount, thereby enabling the mass-production of biodegradable polymer microspheres.

The emulsion may include at least one of polyvinyl alcohol, polyoxyethylene sorbitan and salts thereof, soybean lecithin, and monoglyceride.

The emulsion may include a surfactant. Anionic, cationic, or amphoteric surfactants may be all used as the surfactant. The surfactant may include, for example, at least one of polyoxyethylene sorbitan monolaurate (Tween 20), polyoxyethylene sorbitan monopalmitate (Tween 40), polyoxyethylene sorbitan monostearate (Tween 60), polyoxyethylene sorbitan monooleate (Tween 80), and polyoxyethylene sorbitan trioleate (Tween 85).

When the emulsion includes polyvinyl alcohol, polyvinyl alcohol may be used by dissolving the polyvinyl alcohol in water or a mixed solution of water and alkyl alcohol. In this case, the polyvinyl alcohol may have an average molecular weight of 50,000 to 200,000. In addition, a content of the polyvinyl alcohol may be included in an amount of 1 to 10 wt% based on the dispersion. Outside the above range, an emulsification of polyvinyl alcohol, which acts as a surfactant, may be weakened, thus making it difficult to make microspheres.

S30: Discharge a mixed solution of the dispersion and the emulsion formed through continuous injection into a stabilization tank.

The emulsion may be continuously supplied to the emulsion tank and may be 1 to 20 times the amount of the dispersion. If the emulsion is less than one time, emulsification may be difficult to occur, and if the emulsion is more than 20 times, productivity may decrease due to excessive use of solvent.

The discharged liquid may be a mixture of the dispersion and the emulsion which are continuously injected, may include the microsphere formed in above S20 and may be continuously discharged to the stabilization tank.

S40: Stabilize the microspheres by a method of evaporating a solvent while slowly stirring the mixed solution collected in the stabilization tank.

As a method of evaporating the solvent, natural evaporation through stirring or decompression evaporation through decompression may be used.

S50: Precipitate the microspheres through solid-liquid separation in the stabilization tank, separate the precipitated microspheres, and repeatedly wash the same several times with the washing solution.

The washing liquid may include at least one of alkyl alcohol, distilled water, or a mixed solution of alkyl alcohol and distilled water.

S60: Collect the microspheres which contain moisture through washing and completely remove the remaining moisture through drying.

The method for drying microspheres may use a freeze-drying method for evenly spreading a cake of microspheres containing moisture, freezing and then drying the same. Alternatively, if the microspheres are made of a highly heat-resistant biodegradable polymer material, the microspheres may be dried over dry heat or naturally.

*The method for preparing biodegradable polymer microspheres according to one embodiment of the present invention may have a uniform particle size, and thus may not require the use of a pulverizer, so that a yield of microspheres having a desired size may be 90% or higher. Further, a size of the microspheres may be easily adjusted by replacing the microspheres with a membrane having a different pore size.

A size of biodegradable polymer microspheres may be 1 to 300 µm. A size of biodegradable polymer microspheres may mean, for example, a particle diameter of biodegradable polymer microspheres.

If the size of biodegradable polymer microspheres is less than 1 pm, there may be concerns over having toxicity in vivo, and if the size of biodegradable polymer microspheres exceeds 300 pm, it may not be suitable for use as an injection formulation.

A method for preparing an injection formulation according to one embodiment of the present invention may refer to FIG. 2 and may include preparing an aqueous solution including biodegradable polymer microspheres (S100), injecting or filling the aqueous solution into a vial or a syringe (S200), cooling the aqueous solution filled in the vial or syringe (S300), freeze-drying the aqueous solution in the vial or syringe (S400), sealing the vial or syringe with a vial cap or a rubber cap (S500), and sterilizing the vial or the syringe (S600).

The method for preparing the injection formulation of the present invention will be described in more detail as follows.

*

S100: Prepare an aqueous solution including biodegradable polymer microspheres. The aqueous solution may include an excipient.

The excipient may include at least one of alginic acid and salts thereof, hyaluronic acid and salts thereof, carboxymethyl cellulose and salts thereof, dextran and salts thereof, collagen, gelatin, and elastin.

A ratio of the biodegradable polymer microsphere and the excipient in the aqueous solution may be 2:8 to 8:2. Outside the above range, it is difficult to evenly disperse the biodegradable polymer microspheres at an appropriate concentration outside the range in which the content of the excipient may be adjusted. In this case, the mixed solution having high viscosity may be evenly dispersed by using a three roll mill or other mixing equipment.

S200: Inject or fill the prepared aqueous solution into a vial or a syringe.

When directly injecting/filling an aqueous solution, the injection/filling device may use a pressurized or nozzle-type device. In addition, when filled in a vial or syringe in a powder state, the aqueous solution including the biodegradable polymer microspheres may be cooled, freeze-dried, pulverized into a powder, and filled in a power form.

S300: Cool the solution injected in the vial or syringe.

When the injected solution is cooled, a temperature may be - 80°C to -10°C. If the temperature is less than -80°C, it may be inappropriate for cooling equipment for production, and if the temperature exceeds -10°C, cooling may not be evenly performed, causing melting or splashing of the injected solution during freeze-drying.

S400: Freeze-dry the cooled solution injected in the vial or syringe.

Freeze-drying may be a process for making a cake-type product with the injected solution, and may fill the pre-lyophilized microspheres in a powder form.

S500: Seal the lyophilized vial or syringe with a vial cap or a rubber cap.

When the vial or syringe is sealed, full stoppering or half-stoppering may be performed according to a sterilization method, and when the sterilization is completed, sealing may be performed through full stoppering.

S600 : Sterilize the sealed vial or syringe.

The sterilization method may be performed through gamma-ray sterilization, e-beam sterilization, ethylene oxide sterilization, or vacuum sterilization.

Hereinafter, a parallel-type continuous reaction device for preparing biodegradable polymer microspheres according to one embodiment of the present invention will be described. Hereinafter, differences between a method for preparing an injection formulation according to one embodiment of the present invention and a method for preparing biodegradable polymer microspheres according to one embodiment of the present invention will be described, and the same or similar things will be omitted.

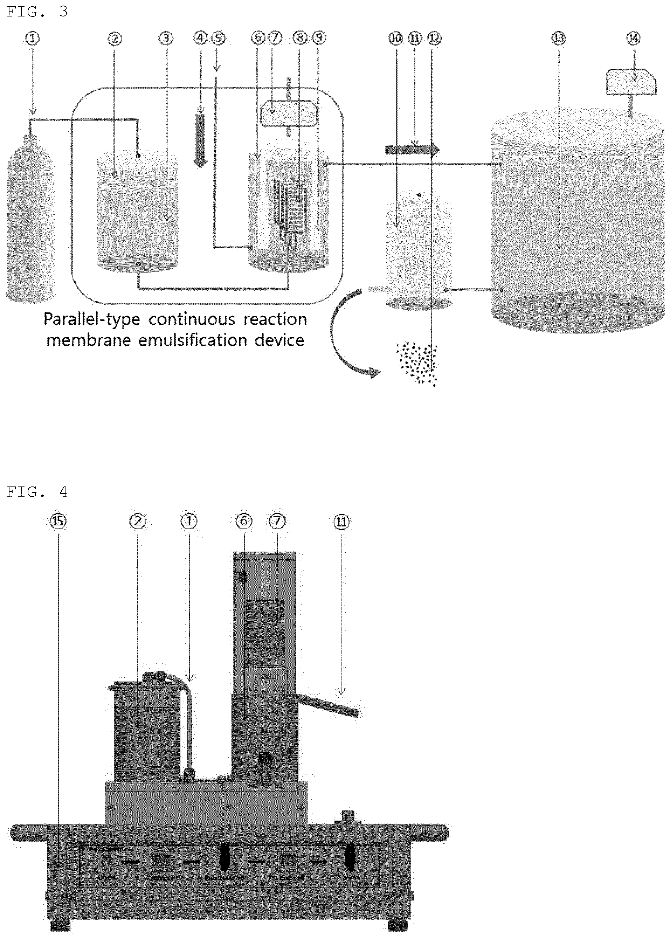

FIG. 3 is a conceptual view showing a parallel-type continuous reaction membrane emulsification device for preparing biodegradable polymer microspheres according to one embodiment of the present invention. FIG. 4 is a view showing a key device in a conceptual view of a parallel-type continuous reaction according to one embodiment of the present invention.

Referring to FIGS. 3 and 4, the parallel-type continuous reaction membrane emulsification device according to one embodiment of the present invention may include a pressurizing tank and a pressurizing line 1, a dispersion tank 2, an emulsion continuous supply line 5, an emulsion tank 6, a stirring motor 7, a parallel-type membrane emulsification module 8, a stirring rod 9, a continuous solid-liquid separating device 10, a discharge port line 11, a stabilization tank 13, a decompression exhaust device 14, and a manipulation part 15.

In the parallel-type continuous reaction membrane emulsification device of the present invention, a dispersion and an emulsion may meet each other through a parallel-type membrane to form biodegradable polymer microspheres having a regular size.

The dispersion may be moved from the sealed dispersion tank 2 to the parallel-type membrane emulsification module 8 by a pressure of air or nitrogen gas, and the dispersion may be slowly ejected through the membrane to meet the emulsion of the emulsion tank 6, thereby forming the biodegradable polymer microspheres. The emulsion may be continuously supplied through the emulsion continuous supply line 5, the dispersion and the emulsion may be mixed by the stirring rod 9, and the mixed solution may be moved to the stabilization tank 13 through the discharge port line 11.

The mixed solution moved to the stabilization tank 13 may stabilize the formed microspheres while the solvent is evaporated and removed by the decompression exhaust device 14, and the formed microspheres may be obtained in a cake state through solid-liquid separation of the continuous solid-liquid separating device 10.

The stirring motor 7 may have a rotation speed of 10 to 500 rpm. If the speed is less than 10 rpm, the emulsion and the dispersion may not be sufficiently stirred, and if the speed is more than 500 rpm, there may be a problem in which the formed microspheres may be broken, and thus the size is changed.

According to the parallel-type continuous reaction membrane emulsification method and device for preparing biodegradable polymer microspheres according to one embodiment of the present invention, spherical particles having a uniform size may be easily prepared, the method and device are suitable for mass production. In addition, there may be an advantage in that the particle size of the biodegradable polymer microspheres may be easily adjusted.

### [Mode for Invention]

Hereinafter, the present invention will be described in more detail through specific examples. The following examples are provided only for the purpose of illustrating the present invention, and the scope of the present invention is not limited thereto.

### Example 1: Conditions for preparing microspheres

10 g of polylactic acid having an average molecular weight of 100,000 was stirred in 100g of trichloromethane to be completely dissolved, and then injected into a dispersion of a parallel-type continuous reaction microsphere preparing device. After the dispersion tank was sealed, nitrogen gas in a pressurizing tank connected to the dispersion tank was pressurized and injected into an emulsion tank through a membrane having a pore size of 30 um for each dispersion flow rate condition in Table 1. An emulsion of 3% PVA (average molecular weight of 130,000) was continuously supplied into the emulsion tank for each flow rate condition in Table 1. The dispersion and the emulsion were mixed well while stirring at 180 rpm. The discharged reaction solution (mixed solution) was continuously received in a stabilization tank and stirred for 24 hours to naturally evaporate trichloromethane, and stirring was stopped to stand for two hours, so that microspheres may be precipitated. After the microspheres were completely precipitated, a filtrate of an upper layer was removed and washed five times by adding 10 L of distilled water, stirring for 30 minutes, precipitating for one hour, and removing a supernatant. After freezing the cake-state microspheres containing moisture, which had been completely washed, the microspheres were subjected to freeze-drying to prepare biodegradable polymer microspheres in a form of powder.

**[Table 1]**

| Experiment sequence number | Emulsion (mL/min) | Dispersion (mL/min) | Condition |
|---|---|---|---|
| 1 | 10 | 5 | Non-uniform particle shape |
| 2 | 20 | 10 | Non-uniform particle shape |
| 3 | 40 | 20 | Clogged membrane |
| 4 | 20 | 5 | Distorted particle shape |
| 5 | 40 | 10 | Distorted particle shape |
| 6 | 80 | 20 | Clogged membrane |
| 7 | 50 | 5 | Slow formation |
| 8 | 100 | 10 | Optimal condition |
| 9 | 200 | 20 | Clogged membrane |

As shown in Table 1, it was confirmed that the most uniform and accurate spherical microspheres might be obtained under the conditions of 10 mL/min of the dispersion and 100 mL/min of the emulsion.

### Example 2: Comparison of parallel-type continuous reaction membrane emulsification method and emulsification solvent evaporation method

According to a general emulsification solvent evaporation method, 1 g of polylactic acid having an average molecular weight of 100,000 was stirred in 10 g of trichloromethane to be completely dissolved, and then mixed with 100 g of an emulsion and rapidly stirred in a homogenizer to prepare microspheres. A stirrer was left alone for two hours so that the microspheres might be precipitated, and then a filtrate of an upper layer was removed after the microspheres were completely precipitated. Washing was performed five times by adding 1 L of distilled water again, stirring for 30 minutes, precipitating for one hour, and removing a supernatant. After freezing the cake-state microspheres containing moisture, which had been completely washed, the microspheres were subjected to freeze-drying to prepare microspheres in a form of powder.

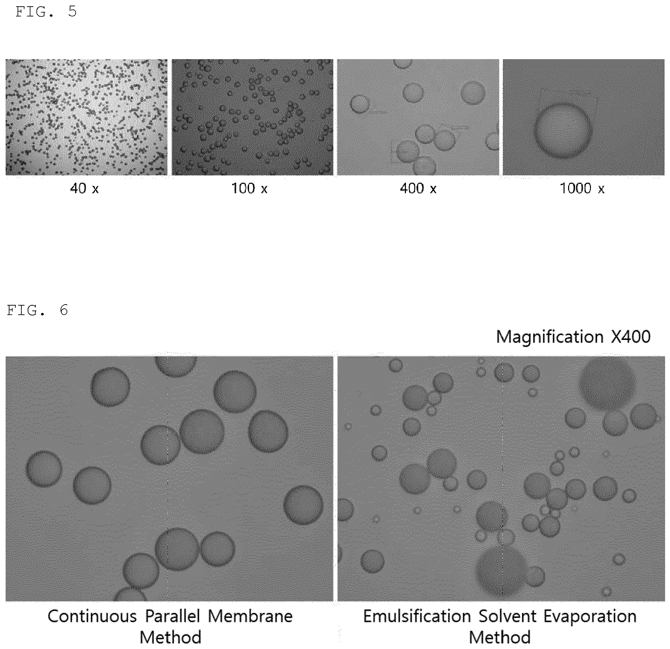

For comparison with the present invention, the microsphere prepared under condition 8 of Example 1 and the microsphere prepared by the general emulsification solvent evaporation method are shown in FIG. 6.

### Example 3: Preparation of microspheres by membrane pore for several biodegradable polymers

Biodegradable polymer microspheres were prepared by using polylactic acid (PLA), polycarprolactone (PCL), and polylactic acid-glycolic acid (PLGA) while changing pores of the membrane under preparation condition 8 of Example 1.

### [Measurement]

FIG. 5 is an electron micrograph of the biodegradable polymer microspheres prepared in Example 1. Referring to FIG. 5, it can be confirmed that perfectly spherical microspheres are formed and are prepared in a sufficiently uniform size without a need for a pulverization process in the present invention.

FIG. 6 is a micrograph comparing microspheres obtained by a parallel-type continuous reaction membrane emulsification method in Example 2 and a general emulsification solvent evaporation method. Referring to FIG. 6, it could be confirmed that the microspheres obtained by the general emulsification solvent evaporation method have a non-uniform particle size, whereas the microspheres obtained by the parallel-type continuous reaction membrane emulsification method according to the present invention have a uniform particle size.

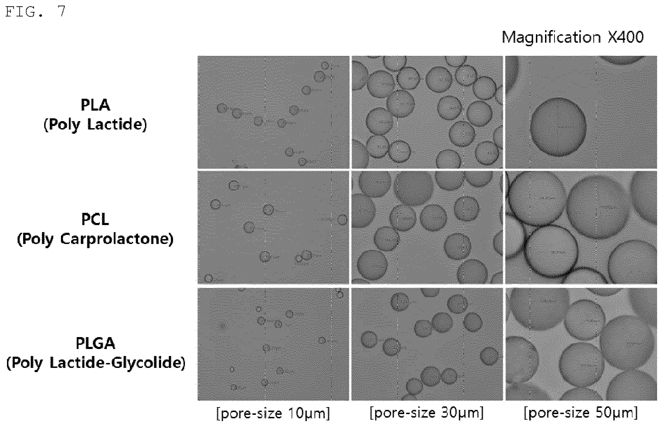

FIG. 7 is a micrograph comparing microspheres prepared in Example 3 by using several biodegradable polymers and membranes having different pores. Referring to FIG. 7, it can be confirmed that a size of the microspheres may be easily adjusted according to a pore of the membrane, and microspheres having a regular particle size distribution in which the size is adjusted under similar conditions for various biodegradable polymers may be prepared.

### <List of reference numbers>

1: Nitrogen tank pressurization line 2: Dispersion tank
3: Dispersion 4: Emulsion continuous supply section
5: Emulsion continuous supply line 6: Emulsion tank
7: Stirring motor 8: Parallel-type membrane emulsification module
9: Stirring rod 10: Continuous solid-liquid separating device
11: Discharged liquid continuous supply section 12: Biodegradable polymer microsphere
13: Stabilization tank 14: Decompression exhaust device
15: Manipulation part

## Claims

1. A method for preparing biodegradable polymer microspheres using a parallel-type continuous reaction membrane emulsification device, the method comprising:
preparing a dispersion in which biodegradable polymers are dissolved;
applying a pressure to the dispersion and slowly injecting the dispersion into an emulsion through a parallel-type membrane to form microspheres;
discharging a mixed solution in which the microspheres are mixed;
receiving the discharged mixed solution to stabilize the microspheres; and
washing and drying the stabilized microspheres, wherein the emulsion is continuously replaced and supplied.

2. The method of claim 1, wherein in the preparing of the dispersion in which the biodegradable polymers are dissolved, the biodegradable polymer is selected from the group including polylactic acid (PLA) and isomers thereof, polyglycolic acid (PGA), polycarprolactone (PCL), polydioxanone (PDO), and polylactic acid-glycolic acid (PLGA) and isomers thereof, and an average molecular weight of the polymer is 20,000 to 300,000.

3. The method of claim 1, wherein the dispersion in which the biodegradable polymers are dissolved includes a solvent, in which the solvent includes at least one of chlorinated hydrocarbon, hydrocarbon, perfluoroalcohol, ethyl ester (EA), ether-based solvent, N,N-dimethyl formamide (DMF), and dimethyl sulfoxide (DMSO), and a content of the biodegradable polymer in the dispersion is 1 to 20 wt%.

4. The method of claim 1, wherein in the forming of microspheres, the formed microspheres have a uniform particle size distribution within 30% of standard deviation in size.

5. The method of claim 1, wherein in the forming of microspheres, the biodegradable polymer microspheres have a size of 1 to 300 µm.

6. The method of claim 1, wherein in the forming of microspheres, a membrane with different pores is used to adjust a microsphere size between 1 and 300 µm.

7. The method of claim 1, wherein the emulsion includes at least one of polyvinyl alcohol, polyoxyethylene sorbitan and salts thereof, soybean lecithin, and monoglyceride.

8. The method of claim 1, wherein the biodegradable polymer microspheres are used as a face forming filler for skin tissue reconstruction and tissue regeneration, a male prosthesis, or a long-lasting injection formulation for urinary incontinence treatment or medical and pharmaceutical uses through drug encapsulation.

9. A method for preparing an injection formulation comprising biodegradable polymer microspheres, the method comprising: preparing an aqueous solution including biodegradable polymer microspheres prepared by any one of claims 1 to 8; injecting the aqueous solution into a vial or a syringe; cooling and freezing the injected aqueous solution, followed by freeze-drying; and sealing and sterilizing the vial or the syringe.

10. The method of claim 9, wherein in the preparing of the aqueous solution including biodegradable polymer microspheres, the biodegradable polymer microspheres are included in an amount of 10 to 80 wt% based on the total weight of the aqueous solution.

11. The method of claim 9, wherein in the preparing of the aqueous solution including biodegradable polymer microspheres, the aqueous solution further includes an additive, and the additive includes at least one of carboxymethyl cellulose and salts thereof, alginic acid and salts thereof, hyaluronic acid and salts thereof, dextran and salts thereof, collagen, gelatin, and elastin, and a content of the additive is 1 to 30 wt% based on the total weight of the aqueous solution.

12. The method of claim 11, wherein the additive is carboxymethyl cellulose, and the biodegradable polymer microspheres are included in an amount of 30 to 60 wt% based on the total weight of the aqueous solution including the carboxymethyl cellulose.

13. The method of claim 9, wherein the biodegradable polymer microspheres have a size of 10 to 300 µm.

14. The method of claim 9, wherein in the sterilizing, gamma-ray sterilization, e-beam sterilization, ethylene oxide sterilization, or vacuum sterilization are performed.

15. An injection formulation comprising biodegradable polymer microspheres and prepared according to the preparation method of any one of claims 9 to 14, wherein the injection formulation is used as a face forming filler for skin tissue reconstruction and tissue regeneration, a male prosthesis, or a long-lasting injection formulation for urinary incontinence treatment or medical and pharmaceutical uses through drug encapsulation.

16. A membrane emulsification device using a parallel-type membrane and a continuous-type reaction, the device comprising:
a dispersion tank for storing a dispersion;
a pressurizing tank and a pressurizing line for pressurizing the dispersion;
an emulsion tank provided with a membrane emulsification module and configured to receive the dispersion;
an emulsion continuous supply line for continuously supplying an emulsion to the emulsion tank;
a discharge port line for discharging a mixture of the dispersion and the emulsion to a stabilization tank;
a stabilization tank for stabilizing microspheres through decompression or evaporation; and
a manipulation part, wherein the membrane emulsification module is a parallel-type membrane emulsification module, in which membranes for injecting the dispersion into the emulsion are arranged in parallel to each other.

17. The membrane emulsification device of claim 16, wherein the dispersion tank is sealed and includes an adjustment device capable of adjusting a pressure from the pressurizing tank, in which the adjustment device adjusts a pressure from the pressurizing tank to 1 kPa to 300 kPa according to a pore size of the membrane.

18. The membrane emulsification device of claim 16, wherein the membrane of the membrane emulsification module is configured such that a plurality of membranes are mounted in parallel to each other or a plurality of reactors respectively equipped with one membrane are provided in parallel to each other.

19. The membrane emulsification device of claim 16, wherein the membrane is replaceable, and thus a size of biodegradable polymer microparticles may be adjusted between 1 and 300 µm through the replacement.

20. The membrane emulsification device of claim 16, wherein the emulsion tank includes a stirring device to evenly disperse the emulsion and the dispersion, which are continuously supplied.

21. The membrane emulsification device of claim 20, wherein the stirring device has a rotational speed of 10 to 500 rpm.
